# EUROPÄISCHE PATENTANMELDUNG

(11) **EP 0 922 469 A2**
(43) Veröffentlichungstag der Anmeldung: **16.06.1999**
(21) Anmeldenummer: 98117119.2
(22) Anmeldetag: 10.09.1998
(51) Int. Cl.: A61N 5/06

(54) **Verfahren zum Durchführen einer Farblichtanwendung**

(30) Priorität: 13.12.1997 DE 19755505
(71) Anmelder: Wella Aktiengesellschaft, 64274 Darmstadt (DE)
(72) Erfinder: Weller, Matthias, 36169 Rosdorf (DE); Rousek, Heinz, 36088 Hünfeld (DE)

(57) **Zusammenfassung**

Bei einem Verfahren zum Durchführen einer Farblichtanwendung, bei dem nachfolgend unterschiedliche Farben in einen Personen aufnehmenden Raum, z. B. eine Sauna, ein Klimabad, oder einen Ruheraum, eingestrahlt werden, kann in einem Gruppenmodus eine Auswahl an ungewünschten Negativfarben getroffen werden. Nach einer derartigen Auswahl erfolgt die Farblichtanwendung ohne die ausgewählte Negativfarbe bzw. die ausgewählten Negativfarben. Dadurch können mehrere Personen eine Farblichtanwendung ohne ihre Negativfarben erhalten.

## Beschreibung

Die Erfindung betrifft ein Verfahren zum Durchführen einer Farblichtanwendung,bei der nacheinanderfolgend unterschiedliche Farben in einen Personen aufnehmenden Raum eingestrahlt werden.

Farblichtbestrahlungen werden bei Personen einerseits für entspannende Zwecke, und andererseits zum Erreichen eines bestimmten Wohlfühlzustandes vorgenommen.

Aus der DE-OS 40 15 406 ist es bekannt, für therapeutische Zwecke Farblicht, z. B. in einer Sauna, auf Personen zu strahlen. Dabei werden neun verschiedene Farben angewendet. Des weiteren sind aus dem DE-G 296 05 685 Programmabläufe für Farblichtanwendungen bekannt, bei denen wechselnde Farben mit veränderten Farbintensitäten einhergehen, um eine verbesserte Farblichtbehandlung durchzuführen.

Die bekannten Verfahren haben den Nachteil, daß sie für den Einsatz bei einer Personengruppe weniger geeignet sind, da in der Regel nicht alle eingesetzten Farben von allen Personen gewünscht werden.

Der Erfindung liegt die Aufgabe zu Grunde, ein Verfahren der eingangs beschriebenen Art zu schaffen, das für quasi alle Personen einer Gruppe geeignet ist.

Gelöst ist die Aufgabe gemäß dem kennzeichnenden Teil des Anspruchs 1. Danach kann in einem Gruppenmodus eine Auswahl von ungewünschten Negativfarben getroffen werden, und nach einer derartigen Auswahl erfolgt eine Farblichtanwendung ohne die ausgewählte Negativfarbe bzw. die ausgewählten Negativfarben.

Das erfindungsgemäße Verfahren hat den Vorteil, daß es für quasi alle Personen einer Gruppe geeignet ist, da jede Person die von ihr nicht gewünschte Farbe (Negativfarbe) bzw. Farben abwählen kann. Die Farbbehandlung erfolgt dann mit einer reduzierten Anzahl verschiedener Farben. Für den Fall, daß alle Farben abgewählt werden, können besondere Vorkehrungen getroffen werden. So kann zur Bildung einer neuen Gruppe aufgefordert werden, oder ein Minimalprogramm kann gestartet werden.

Weitere, vorteilhafte Ausgestaltungen der Erfindung sind in den Ansprüchen 2 bis 13 beschrieben.

Ist eine Umstellung vom Gruppenmodus auf einen Einzelpersonenmodus vorgesehen, und ist zudem im Einzelmodus entweder eine Auswahl von Negativfarben oder eine Auswahl von Positivfarben vorgesehen (Anspruch 2), so kann die Farbtherapie wahlweise für eine Gruppe oder eine Einzelperson angewendet werden. Eine Einzelperson kann Positiv- oder Negativfarben auswählen, je nachdem, ob eine Eingabe gewünschter oder ungewünschter Farben sinnvoller ist.

Sofern keine Auswahl getroffen wurde, kann ein Grundprogramm für einen Farbwechsel ablaufen (Anspruch 3). Es könnte aber auch eine Unterscheidungsmöglichkeit für unterschiedliche Grundprogramme vorgesehen sein.

Das in Anspruch 4 beschriebene Grundprogramm wurde von Testpersonen überaus positiv bewertet. Es entspricht einem natürlichen Farbenbedürfnis der Testpersonen.

Unterschiedliche, insbesondere aufwendige Farbzyklen können in einfacher Weise ausgewählt und abgerufen werden, wenn analog Anspruch 5 Chipkarten vorgesehen sind, auf denen ein Farbmodus gespeichert ist, der gelesen und ausgeführt wird. Die Information einer Chipkarte kann außer einer Farbreihenfolge auch einen Musikablauf (Anspruch 7) und eine Beleuchtungsstärke vorgeben.

Wird monochromatisches Licht in den Raum eingestrahlt (Anspruch 6), so ist der Anwendungserfolg größer als bei nicht monochromatischem Licht.

In vorteilhafter Weise kann die Intensität einer Farbe bzw. deren Strahlungsleistung in Abhängigkeit der Temperatur und/oder der Luftfeuchtigkeit des Raumes geregelt werden (Anspruch 8). Hierdurch wird dem Rechnung getragen, daß das Empfinden einer Farbintensität von diesen Parametern abhängt. Eine Anwendung dieser Abhängigkeit ist insbesondere für Saunen, Klimabäder und Ruheräume geeignet (Anspruch 12).

Werden die zur Verfügung stehenden Farben an einem Steuergerät identisch zu den tatsächlich einzustrahlenden Farben angezeigt (Anspruch 9), so erhält ein Farblichtanwender im Auswählzeitpunkt eine optimale Information über die zum Einsatz kommenden Farben. Die Anzeige jeder Farbe in einer Leuchtfläche mit einer anderen Geometrie als die restlichen Farben (Anspruch 10) unterstützt den Auswählprozeß.

Jeder Anwender kommt unabhängig von seiner Position im Anwendungsraum in den gleichen Farblichteinfluß, wenn gemäß Anspruch 11 die Strahlung gleichmäßig in den gesamten Raum gestreut wird.

Um auch in dem Fall eine von Gruppenmitgliedern beeinflußte Farbauswahl zu treffen, wenn alle Farben oder alle Farben bis auf eine abgewählt wurden, wird analog Anspruch 13 vorgeschlagen, daß keine weitere Auswahl mehr erfolgen kann, wenn nur noch eine Farbe nicht ausgewählt wurde, und daß die Farbbehandlung lediglich mit dieser Farbe durchgeführt wird. Hierbei wird auch dann noch eine Farbe ausgewählt, wenn alle Farben als Negativfarben bestimmt wurden, nämlich die zuletzt angewählte Farbe.

Im folgenden wird die Erfindung an Hand eines Ausführungsbeispieles näher erläutert. Es zeigt:

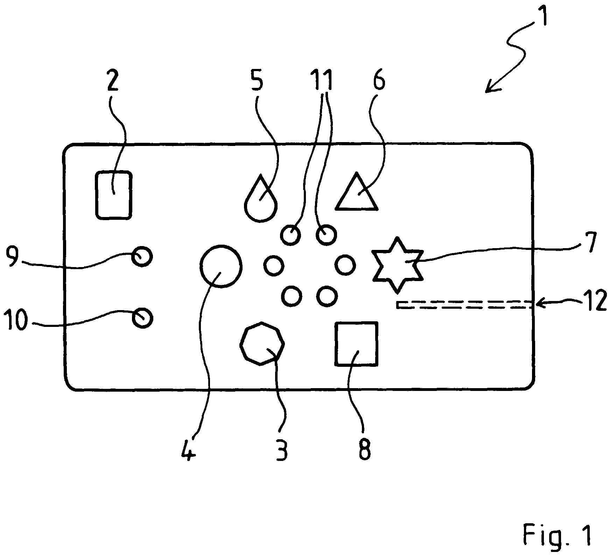

Figur 1 in einer Seitenansicht ein Steuergerät zum Auswählen der für eine Farblichtanwendung zur Verfügung stehenden Farben.

Ein Steuergerät 1 ist zur Steuerung einer Farblichtanwendung an einer Sauna vorgesehen. Nach Betätigung eines Ein/Aus-Schalters 2 werden sechs Farben am Steuergerät 1 angezeigt. Die Farbe violett wird von einer beleuchteten Farbanzeige 3 in Form eines Achtecks angezeigt. Die Farbe blau erscheint in einer Farbanzeige 4 in Form eines Kreises. Die weiteren Farbanzeigen 5, 6, 7, 8 zeigen die Farben grün, gelb, orange und rot. Wird keinerlei Farbauswahl von den Saunagängern getroffen, so läuft ein Grundprogramm für einen Farblichtwechsel in der Sauna an. Mit diesem Grundprogramm werden innerhalb von acht Minuten sämtliche Farben jeweils einmal angewählt. Der Farbzyklus beginnt bei violett und wechselt über blau, grün, gelb und orange zu rot. Im Grundprogramm sind drei derartige Farbzyklen vorgesehen, wobei ein Farbzyklus in relative Zeitintervalle eingeteilt ist. Die relativen Zeitintervalle sind derart bestimmt, daß für violett 23 %, blau 27 %, grün 15 %, gelb 9 %, orange 11 % und rot 15 % der Gesamtdauer für einen Farbzyklus vorgesehen sind. Die Farben werden mittels monochromatischen Lichtes gleichmäßig in den gesamten Saunaraum eingestrahlt.

Die Farbanzeigen 3, 4, 5, 6, 7, 8 zeigen die tatsächlich in den Saunaraum eingestrahlten Farben identisch an. Dabei wird jede Farbe in einer Leuchtfläche mit einer anderen Geometrie angezeigt.
Die Intensität der Strahlung wird vom Steuergerät in Abhängigkeit der Saunatemperatur geregelt.

Es ist möglich, durch Betätigung eines Gruppenmodusschalters 9 ein Farbauswahlverfahren für eine Gruppe von Saunagängern, oder durch Betätigung eines Schalters 10 einen Modus für Einzelpersonen zu starten.

Im Gruppenmodus kann eine Auswahl an ungewünschten Negativfarben getroffen werden. Nach einer derartigen Auswahl erfolgt eine Farblichtanwendung ohne die ausgewählte Negativfarbe bzw. die ausgewählten Negativfarben.

Im Gruppenmodus wird eine Farbe abgewählt, indem ein zu einer Farbanzeige 3, 4, 5, 6, 7, 8, 9 gehörender Tastpunkt 11 berührt wird. Würden zwei Farben abgewählt, so erfolgte eine Farblichtanwendung mit den restlichen vier Farben.

Um noch eine Farblichtanwendung in dem Fall durchzuführen, daß mehrere Personen nacheinander alle Farben abwählen wollen, ist vorgesehen, daß keine weitere Auswahl mehr erfolgen kann, wenn nur noch eine Farbe nicht ausgewählt wurde. Mit dieser Farbe wird sodann eine Farblichtbehandlung durchgeführt.

Durch Berühren des Schalters 10 erfolgt ein Umschalten vom Gruppenmodus zu einem Einzelpersonenmodus. In diesem Modus ist eine Auswahl von Positivfarben vorgesehen. Eine einzelne Person kann hierbei die von ihr für eine Farblichtanwendung gewünschten Farben durch Berühren der entsprechenden Tastpunkte 11 wählen.

Durch Einführen einer Chipkarte in einen Schlitz 12 kann ein auf der Chipkarte gespeicherter Farbmodus vom Steuergerät 1 gelesen und für eine Farblichtanwendung ausgeführt werden. Auf der Chipkarte sind auch Daten für eine Anwahl unterschiedlicher Tracks eines CD-Spielers gespeichert, so daß zu dem Farbmodus ein passender Musikablauf gestartet wird.

## Patentansprüche

1. Verfahren zum Durchführen einer Farblichtanwendung, bei der nachfolgend unterschiedliche Farben in einen Personen aufnehmenden Raum eingestrahlt werden, dadurch gekennzeichnet, daß in einem Gruppenmodus eine Auswahl an ungewünschten Negativfarben getroffen werden kann, und daß nach einer derartigen Auswahl eine Farblichtanwendung ohne die ausgewählte Negativfarbe bzw. die ausgewählten Negativfarben erfolgt.

2. Verfahren nach Anspruch 1, dadurch gekennzeichnet, daß eine Umstellung vom Gruppenmodus auf einen Einzelpersonenmodus vorgesehen ist, und daß im Einzelmodus entweder eine Auswahl von Negativfarben oder eine Auswahl von Positivfarben vorgesehen ist.

3. Verfahren nach Anspruch 1 oder Anspruch 2, dadurch gekennzeichnet, daß ein Grundprogramm für einen Farbwechsel abläuft, sofern keine Auswahl getroffen wurde.

4. Verfahren nach Anspruch 3, dadurch gekennzeichnet, daß ein Farbzyklus im Grundprogramm in relative Zeitintervalle eingeteilt ist, wobei für violett 23 %, blau 27 %, grün 15 %, gelb 9 %, orange 11 % und rot 15 % der Gesamtdauer des Farbzyklusses vorgesehen sind, und der Farbzyklus diese Farben in dieser Reihenfolge enthält.

5. Verfahren nach Anspruch 1 oder Anspruch 2, dadurch gekennzeichnet, daß ein auf einer Chipkarte gespeicherter Farbmodus gelesen und ausgeführt wird.

6. Verfahren nach mindestens einem der vorgenannten Ansprüche, dadurch gekennzeichnet, daß monochromatische Farben in den Raum eingestrahlt werden.

7. Verfahren nach mindestens einem der vorgenannten Ansprüche, dadurch gekennzeichnet, daß ein programmgesteuerter Musikablauf vorgesehen ist.

8. Verfahren nach mindestens einem der vorgenannten Ansprüche, dadurch gekennzeichnet, daß die Intensität bzw. die Strahlungsleistung in Abhängigkeit der Temperatur und/oder der Luftfeuchtigkeit des Raumes geregelt wird.

9. Verfahren nach mindestens einem der vorgenannten Ansprüchen, dadurch gekennzeichnet, daß an einem Steuergerät die zur Verfügung stehenden Farben identisch zu den tatsächlich einzustrahlenden Farben angezeigt. werden.

10. Verfahren nach Anspruch 1 oder Anspruch 9, dadurch gekennzeichnet, daß jede Farbe in einer Leuchtfläche mit einer anderen Geometrie angezeigt wird.

11. Verfahren nach Anspruch 1, Anspruch 2, Anspruch 3 oder Anspruch 4, dadurch gekennzeichnet, daß die Strahlung gleichmäßig in den gesamten Raum gestreut wird.

12. Verfahren nach Anspruch 1 oder Anspruch 8, dadurch gekennzeichnet, daß als Raum eine Sauna, ein Klimabad oder ein Ruheraum vorgesehen ist.

13. Verfahren nach Anspruch 1, dadurch gekennzeichnet, daß keine weitere Auswahl mehr erfolgen kann, wenn nur noch eine Farbe nicht ausgewählt wurde, und daß die Farbbehandlung mit dieser Farbe durchgeführt wird.
